# EUROPEAN PATENT APPLICATION

(11) **EP 4 696 772 A1**
(43) Date of publication of application: **18.02.2026**
(21) Application number: 24789122.9
(22) Date of filing: 12.04.2024
(51) Int. Cl.: C12N 5/078, C12N 5/0786, A61K 35/15

(54) **COMPOSITION FOR INDUCING DIFFERENTIATION IN ERYTHROID PRECURSOR CELLS**

(30) Priority: 13.04.2023 KR 20230048865; 24.11.2023 KR 20230165958
(71) Applicant: IUCF-HYU (Industry-University Cooperation Foundation Hanyang University), Seoul 04763 (KR)
(72) Inventor: BAEK, Eun Jung, Seoul 06717 (KR); PARK, Sung Ah, Seoul 05256 (KR)
(74) Representative: dompatent
(86) International application number: PCT/KR2024/095705
(87) International publication number: WO 2024/215182

(57) **Abstract**

The present invention relates to a composition for inducing differentiation in erythroid precursor cells and, more specifically, to: a composition comprising macrophage-derived extracellular vesicles for inducing differentiation of erythroid precursor cells; and a method for inducing differentiation into erythrocyte by treating erythroid precursor cells with macrophage-derived extracellular vesicles.

## Description

### [Technical Field]

The present invention relates to a composition for inducing differentiation of erythroid precursor cells, which includes macrophage-derived extracellular vesicles.

The present application claims priority and the benefit of Korean Patent Application No.10-2023-0165958, filed on November 24, 2023 in the Korean Intellectual Property Office, the entire disclosure of which is incorporated herein by reference.

### [Background Art]

Blood transfusion is an important therapeutic approach in emergency situations and chronic hematologic disorders (such as anemia, myelodysplastic syndrome, and leukemia). However, due to the issue of hyperimmunization caused by multiple transfusions and the anticipated decrease in the number of eligible blood donors compared to demand in light of the aging worldwide population, transfusion issues are expected to become an important public health concern in the future. Currently, research on *in vitro* erythrocyte generation is being conducted according to stem cell type, but further investigation into the normal production of erythrocytes is still needed because the efficiency of proliferation, differentiation, and enucleation differs from *in vivo* efficiency.

Meanwhile, all cells exchange information with other cells or the external environment, and to facilitate such information exchange, cells secrete various substances into the external environment, and representative soluble factors include cytokines, chemokines, hormones, and neurotransmitters. Recently, other than the soluble factors, information exchange via extracellular vesicles (EVs) is gradually receiving attention. EVs are nano-scale vesicles surrounded by a lipid bilayer, which are secreted into the external environment by all cells.

Extracellular vesicles (EVs) contain proteins, lipids, various types of RNA and DNA, and may be relatively easily isolated from various biofluids such as blood, cerebrospinal fluid (CSF), urine, amniotic fluid, breast milk, saliva, and semen. In EVs, a specific protein is highly concentrated, and thus is easily detected. These EVs contain various different components depending on the type of cells from which they originated, and the analysis of vesicle components can be utilized as biomarkers for early diagnosis of diseases or as indicators of patient response to treatment. In addition, EVs themselves can be used as carriers for a therapeutic agent. Due to these characteristics and applicability, research on EVs has been accelerating recently.

Particularly, macrophage-derived EVs are known to play a pivotal role as a mediator in the tumor microenvironment (TME), and show the capability of promoting communication between cells and between organs. This is largely due to their ability to carry various types of bioactive molecules, including nucleic acids and proteins, which are essential for these communication processes.

Thus, although research on EVs has recently attracted attention, there have been no studies yet on the effects of EVs on erythropoiesis. In addition, although it is known that the interaction between macrophages and erythroid precursor cells in the erythroblastic island within the bone marrow is important, the underlying mechanism has not yet been clearly elucidated.

Existing studies aiming to differentiate erythrocytes *in vitro* have limitations in yield. Accordingly, the inventors conducted extensive research to improve erythrocyte differentiation efficiency and discovered that macrophage-derived EVs promote the differentiation of erythroid precursor cells and regulate the erythrocyte formation process, leading to the present invention.

### [Detailed Description of Invention]

### [Technical Problem]

The present invention is directed to providing a composition for inducing differentiation of erythroid precursor cells, which includes macrophage-derived EVs.

The present invention is also directed to providing a method of inducing differentiation of erythroid precursor cells, which includes culturing erythroid precursor cells in a medium containing macrophage-derived EVs.

### [Technical Solution]

The inventors made efforts to produce erythrocytes *in vitro* and improve the yield of *in vitro* erythrocyte differentiation to resolve the shortage of blood supply needed for surgical operations or other purposes. As a result, when erythroid precursor cells were treated with macrophage-derived EVs, it was found that the EVs can promote differentiation of erythroid precursor cells into erythrocytes, and regulate the erythropoiesis.

Accordingly, the present invention provides a composition for inducing differentiation of erythroid precursor cells, which includes macrophage-derived EVs. The composition for inducing differentiation of the present invention can be used to induce differentiation and maturation of erythroid precursor cells into erythrocytes and regulate the erythropoiesis process.

In the present invention, "progenitor cells" are undifferentiated cells with self-renewal and differentiation capacity, but are ultimately differentiated into a predetermined type of cells. Progenitor cells have predetermined differentiation pathways but generally do not express markers of mature, fully differentiated cells, or do not function as mature, fully differentiated cells. Accordingly, the progenitor cells differentiate into related cell types but generally cannot form a wide variety of cell types under normal conditions. In the present invention, erythroid precursor cells are used.

In the present invention, "erythroid precursor cells" refer to all cells involved in erythropoiesis, excluding fully mature erythrocytes, for example, hematopoietic stem cells or erythroid cells before enucleation. For example, the erythroid precursor cells may be CD34⁺ cells. In addition, the erythroid precursor cells may be selected from the group consisting of burst-forming unit-erythroids (BFU-Es), colony-forming unit-erythroids (CFU-Es), proerythroblasts, basophilic erythroblasts, polychromatic erythroblasts, orthochromatic erythroblasts, and reticulocytes. The erythroid precursor cells may be obtained from various sources, such as peripheral blood, umbilical cord blood, or bone marrow.

In the present invention, "extracellular vesicle (EV)" refers to a nanoscale membrane structure produced and secreted by various cell types depending on the physiological or pathological state of the cell. These vesicles include various biological molecules produced by cells, and play an important role in intercellular communication by transmitting information. Particularly, these vesicles are surrounded by a lipid bilayer derived from the cell membrane and may contain proteins, lipids, RNA, DNA, and other metabolites. These EVs may be classified into exosomes, ectosomes/microvesicles, and the like depending on their biogenesis mechanisms.

In the present invention, "macrophages" as members of the immune system, function as part of the non-specific defense mechanism, regulate inflammatory responses, and secrete important cellular cytokines and chemoattractants that promote tissue repair and regeneration. These secretory substances activate other nearby immune cells or participate in intercellular signaling, thereby adjusting the intensity and duration of the immune response. In addition, macrophages possess an antigen-presenting function, and function to display an antigen derived from a pathogen on their cell surface and activate specific immune cells such as T cells.

Upon tissue damage or infection, macrophages may transition to two major activation states, generally called M1 and M2. M1 macrophages are pro-inflammatory macrophages that promote antibacterial and tumoricidal responses, while M2 macrophages are involved in promoting anti-inflammatory responses and tissue repair.

In the present invention, "macrophage-derived EVs" may refer to EVs secreted by macrophages, and include a biologically active material associated with the functional characteristics of macrophages.

In one embodiment of the present invention, the macrophage-derived EVs may be derived from M2 macrophages. Accordingly, the present invention discloses the effect of EVs secreted particularly by M2 macrophages on erythropoiesis.

In the present invention, "differentiation" refers to the process in which cells, structures, or tissues lacking a specific function or morphology divide and grow to obtain a mature structure or specific function. For instance, in the present invention, it refers to the process where an immature cell develops into a mature erythrocyte. In addition, in the present invention, "erythropoiesis" refers to a process of developing mature erythrocytes from hematopoietic stem cells, and in the erythropoiesis process, the growth, differentiation and maturation of erythroid precursor cells occur.

In the present invention, the macrophage-derived EVs may induce differentiation and maturation of erythroid precursor cells into erythrocytes and regulate erythropoiesis. In addition, in the present invention, the macrophage-derived EVs may regulate various factors, for example, cell cycle, a differentiation rate, hormones, iron availability, the composition and structure of the cell membrane, or the intracellular and extracellular environments in the erythropoiesis and differentiation processes.

In the present invention, the macrophage-derived EVs may induce the differentiation of erythroid precursor cells and regulate erythropoiesis through a direct interaction between cells by the contact with erythroid precursor cells. The macrophage-derived EVs may induce the differentiation of erythroid precursor cells through direct internalization into erythroid precursor cells, or by activating intracellular signaling pathways via cell adhesion molecules, but the present invention is not limited thereto. The macrophage-derived EVs may induce the differentiation of erythroid precursor cells through intracellular signaling or the regulation of gene expression via a non-contact manner with erythroid precursor cells, but the present invention is not limited thereto.

As a result of observing the EVs secreted from the macrophages after being labeled with fluorescence, it was confirmed that the EVs are directly internalized into the erythroid precursor cells.

In addition, in the present invention, it was confirmed that the macrophage-derived EVs increase the expression of cell adhesion molecules. In the present invention, "cell adhesion molecule (CAM)" or "adhesion protein," as a protein present on a cell surface, refers to a molecule that mediates adhesion between cells, or between a cell and an extracellular matrix, and plays a critical role in biological processes, such as cell-cell interactions, cell migration, inflammatory responses, wound healing, tissue formation, immune responses, and metastasis.

Particularly, in the present invention, as a result of confirming the expression of erythroblastic island-associated adhesion proteins, such as macrophage erythroblast attacher (MAEA), intercellular adhesion molecule 4 (ICAM4), the erythropoietin receptor (EPOR), and alpha hemoglobin stabilizing protein (AHSP), it was confirmed that the expression of the adhesion proteins increased in erythroid precursor cells treated with macrophage-derived EVs, as compared to a control.

In the present invention, MAEA or erythroblast macrophage protein (EMP) is a protein that mediates interactions between erythroid precursor cells and macrophages, and promotes the adhesion of erythroid precursor cells to central macrophages within erythroblastic islands, thereby playing a critical role in the maturation and differentiation of erythrocytes. ICAM4 is an adhesion molecule found on the surface of erythrocytes that mediates the adhesion between erythrocytes and endothelial cells, and regulates the migration of erythrocytes in blood vessels and into tissues. EPOR is a receptor located on the surface of erythroid precursor cells, and it binds to erythropoietin (EPO), a hormone that regulates erythrocyte production and it activates signaling pathways that promote the survival, maturation, and differentiation of erythroid precursor cells. AHSP binds to alpha-globin, thereby reducing the toxicity of globin chains that cannot transport oxygen on their own, and protects them before they are assembled into hemoglobin. Therefore, it plays a critical role in the efficient production and maturation of erythrocytes. The above-mentioned adhesion protein MAEA, ICAM4, EPOR, or AHSP corresponds to an adhesion protein expressed during the differentiation stage of erythroid precursor cells, and it is possible to use the increased expression of these adhesion proteins to determine whether erythroid precursor cell differentiation is induced. For example, these adhesion proteins can be expressed during differentiation from proerythroblasts to basophilic erythroblasts.

In one embodiment of the present invention, as a result of confirming whether the expression of the adhesion proteins increased by treating macrophage-derived EVs during the proliferation of erythroid precursor cells, it was confirmed that the expression of ICAM4 and AHSP was significantly increased in the EV-treated groups compared to the control. Therefore, the composition for inducing differentiation including the macrophage-derived EVs of the present invention can be effectively used for inducing erythroid precursor cell differentiation.

In addition, in one embodiment of the present invention, when erythroid precursor cells were treated with macrophage-derived EVs, the enucleation rate of the erythroid precursor cells increased, and the expression of GPA and CD71, protein markers expressed in mature erythrocytes, increased. Therefore, the composition for inducing differentiation including the macrophage-derived EVs of the present invention can be effectively used in the differentiation and maturation of erythroid precursor cells.

The diameter of the macrophage-derived EVs included in the composition of the present invention may be 50 to 1000 nm, for example, 50 to 800 nm, 50 to 700 nm, 50 to 600 nm, 50 to 500 nm, 50 to 400 nm, 50 to 300 nm, 50 to 250 nm, 100 to 250 nm, 100 to 230 nm, 150 to 230 nm, or 200 to 230 nm.

In addition, in the present invention, the macrophage-derived EVs may be included in the composition at a concentration of 1 x 10¹ to 1 x 10⁴ EVs/cell, 5 x 10¹ to 1 x 10⁴ EVs/cell, or 5 x 10¹ to 2 x 10³ EVs/cell.

In the present invention, erythroid precursor cells may differentiate into erythrocytes by treating the erythroid precursor cells with the composition for inducing differentiation including the macrophage-derived EVs and culturing them in a differentiation and maturation medium. The medium used in the present invention may be a cell culture minimum medium (CCMM) containing only carbon, nitrogen and trace elements, as a medium for obtaining erythrocytes from erythroid precursor cells *in vitro.* That is, it is a basal medium, a mixture containing essential sugars, amino acids, and water required for cells to live, excluding serum, nutrients, and various growth factors. For example, the cell culture minimum medium may be Dulbecco's modified Eagle's medium (DMEM), endothelial differentiation medium (EDM), minimal essential medium (MEM), basal medium Eagle (BME), RPMI 1640, F-10, F-12, α-minimal essential medium (α-MEM), Glasgow's minimal essential medium (G-MEM), or Iscove's modified Dulbecco's medium.

In addition, the medium used in the present invention may include vitamin C that can maintain stability against oxidative stress *in vitro,* and if needed, may further include an antibiotic such as penicillin, streptomycin, or gentamycin.

The medium used in the present invention may further include at least one component selected from the group consisting of stem cell factor (SCF), IL-1, IL-3, IL-4, IL-5, IL-11, granulocyte macrophage-colony stimulating factor (GM-CSF), macrophage colony-stimulating factor (M-CSF), granulocyte-colony stimulating factor (G-CSF), and erythropoietin (EPO) to promote the differentiation and maturation of erythroid precursor cells. More preferably, the medium used in the present invention includes hydrocortisone, SCF, IL-3, and EPO in the differentiation stage from a proerythroblast to a basophilic erythroblast; SCF, IL-3 and EPO in the differentiation stage from a basophilic erythroblast to a polychromatic erythroblast; and EPO in the differentiation stage from a polychromatic erythroblast to an orthochromatic erythroblast. However, this medium does not include a cytokine in the differentiation stage (that is, enucleation) from an orthochromatic erythroblast to a polychromatic erythrocyte (that is, a reticulocyte).

The culture environment may be any environment that is appropriate for inducing the differentiation of erythroid precursor cells *in vitro.* For example, the culture may be performed under conditions of 5% CO₂ and 36 to 38 °C, and preferably, 37 °C.

In one embodiment of the present invention, the erythroid precursor cells treated with the macrophage-derived EVs may be co-cultured with stromal cells. The stromal cells may be mesenchymal stem cells (MSCs), MS-5 cells, or OP-9 cells, but the present invention is not limited thereto. In another embodiment of the present invention, the erythroid precursor cells treated with the macrophage-derived EVs may differentiate alone in the medium without the co-culture with stromal cells. For example, when the erythroid precursor cells differentiate alone in the medium, an additional process of isolation from the co-cultured cells is not necessary, so it is possible to independently induce erythrocyte differentiation in an *in vitro* state without a separate cell isolation or filtering process.

In addition, the present invention provides a method of inducing erythroid precursor cell differentiation, which includes culturing erythroid precursor cells in a medium containing macrophage-derived EVs.

All of the content related to the composition for inducing differentiation can be directly applied mutatis mutandis to the method for inducing erythroid precursor cell differentiation.

The present invention provides erythrocytes that are differentiated and matured by the method for inducing erythroid precursor cell differentiation.

### [Advantageous Effects]

The present invention provides a method for inducing differentiation into erythrocytes by treating erythroid precursor cells with a composition for inducing differentiation of erythroid precursor cells including macrophage-derived EVs, or the macrophage-derived EVs. Therefore, the present invention can meet the clinical demand for blood for transfusion by providing a novel method for *in vitro* production or differentiation of erythrocytes.

### [Description of Drawings]

FIG. 1 show schematic diagrams illustrating medium protocols used in the polarization of macrophages and the proliferation and differentiation of erythroid precursor cells, respectively (FIG. 1A: M2 polarization of macrophages induced to M0 from human monocytic THP-1 cells. FIG. 1B: umbilical cord red blood cell proliferation protocol using EVs. FIG. 1C: umbilical cord red blood cell differentiation protocol using EVs).
FIG. 2A shows histograms that show the EV particle size distribution of isolated THP-1 derived M2 macrophage supernatants.
FIG. 2B shows images of EV distribution in PBS (video screenshots recorded using Malvern NanoSight LM10 (top) and NanoSight NTA 3.4 (bottom)).
FIG. 2C is a graph illustrating the average size and mode distribution of EVs of THP-1 derived macrophages.
FIG. 3 shows graphs obtained by measuring the expression of M2 markers (VEGFA, CD169, CD163, and STAT6) and specific adhesion factors (VCAM1 and EMP) at erythroblastic islands through quantitative real-time PCR.
FIG. 4A is a set of CLSM images of erythrocytes (green) and EVs (red) at 24 hours after treatment with EVs.
FIG. 4B shows the migration of erythrocytes (green) and EVs (red) using live cell imaging 1 hour after treatment with EVs.
FIG. 5 shows the growth rate, aggregation and viability of cells, confirmed after treating erythroid precursor cells with EVs according to the present invention at different concentrations.
FIG. 6 are graphs showing the expression patterns of erythroblastic island-associated adhesion proteins, EMP, ICAM4, EPOR and AHSP, confirmed 24 and 72 hours after treating erythroid precursor cells with EVs according to the present invention at different concentrations.
FIG. 7 shows the GPA and CD71 expression confirmed through flow cytometry after treating erythroid precursor cells with EVs according to the present invention at different concentrations.
FIG. 8 shows GPA and CD49d expression, confirmed through flow cytometry after treating erythroid precursor cells with EVs according to the present invention at different concentrations.
FIG. 9A shows the expression of GPA and CD71 converted into a mean fluorescence intensity (MFI) histogram after treating erythroid precursor cells with EVs according to the present invention at different concentrations.
FIGS. 9B and 9C show the comparison between the morphology and maturation status of cells on day 2 and day 4 of differentiation following Wright-Giemsa staining, after treating erythroid precursor cells with EVs according to the present invention at different concentrations (in FIG. 9B, the pink arrow indicates a polychromatic erythroblast, and a red arrow indicates a reticulocyte).

### [Best Mode]

Hereinafter, the present invention will be described in detail with reference to the following examples. The following examples are merely provided to exemplify the present invention, and the contents of the present invention are not limited to the following examples.

### [Examples]

### 1. Preparation of M2 macrophage-derived EVs

### 1) Culture of M2 macrophages and EV collection

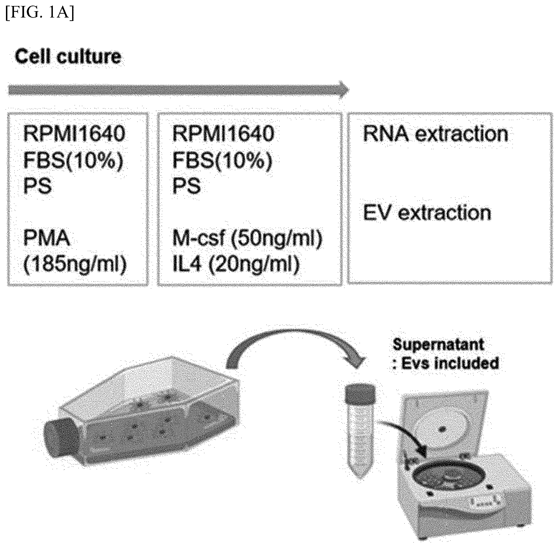

To consistently produce macrophage-derived EVs, human monocytic THP-1 cells were induced into M0 macrophages and then polarized to M2. First, 4 x 10⁵ cell/mL of THP-1 cells were differentiated into M0 macrophages for 24 hours in a RPMI-1640 medium (Gibco, Roswell Park Memorial Institute: RPMI 1640) supplemented with heat-inactivated 10% fetal bovine serum (FBS), 1% penicillin-streptomycin, and 185 ng/mL phorbol 12-myristate 13-acetate (PMA), and then the M0 macrophages were treated with RPMI 1640 supplemented with 50 ng/mL human recombinant M-CSF and 20 ng/mL human recombinant IL-4 for 48 hours to be polarized to M2 (FIG. 1A).

After the human monocytes THP-1 were induced to M2 and cultured in a RPMI-1640 culture medium for 24 hours, a supernatant was collected, followed by isolating EVs. To remove cell debris from the supernatant, the cell culture was centrifuged at 3000 g for 15 minutes, the supernatant was treated with ExoQuick^{™}-TC (System Biosciences) for at least 12 hours at 4 °C according to the manufacturer's protocol and then isolated, diluted with PBS, and stored at -80 °C.

### 2) Analysis of M2 macrophage-derived EVs

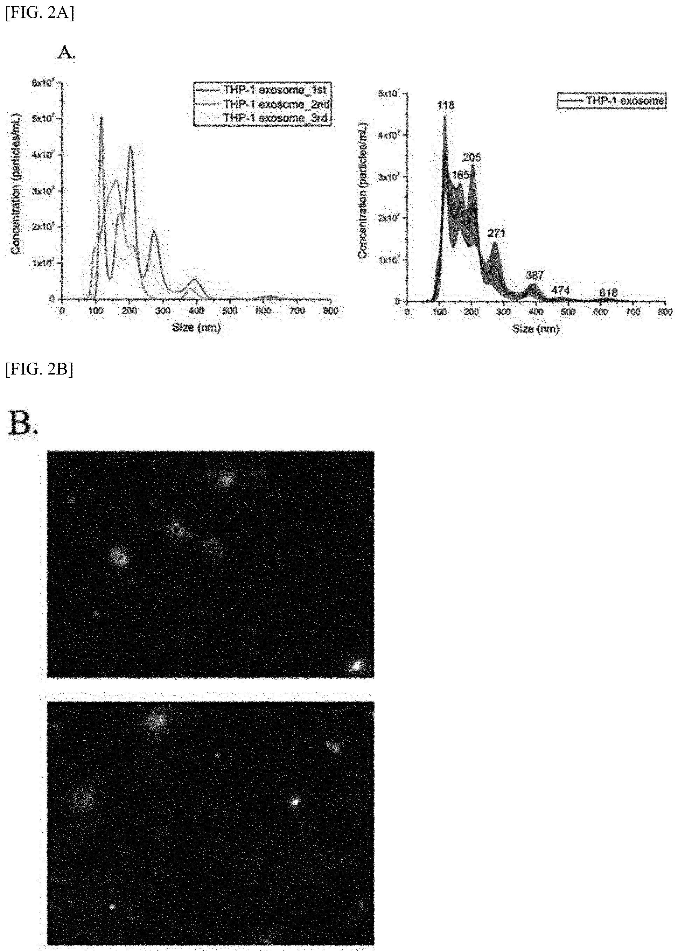

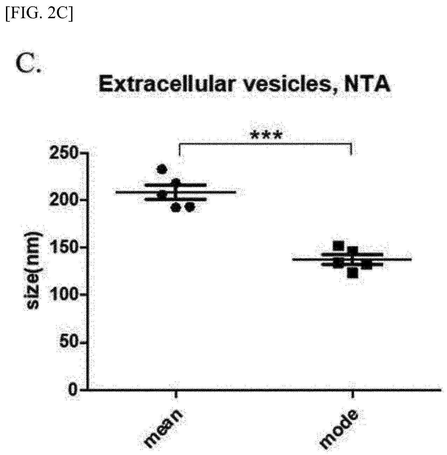

The size, distribution, and concentration of the isolated THP-1 M2 macrophage-derived EVs were confirmed through nanoparticle tracking analysis (NTA) (FIG. 2).

First, after some EVs were introduced to NTA, the Brownian motion of particles was measured on each of the x, y and z axes, and it was confirmed that exosomes were generally distributed between 150 to 250 nm. As a result of NTA analysis for some EVs from each sample, the average size was 208 nm and the mode was 137.62 nm (student t-test; ***p <0.0001). The NTA size distribution analysis showed that the average of the mode values was between 100 to 150 nm, indicating that exosomes were predominantly present, with some other forms of EVs, such as ectosomes or microvesicles, also being present.

### 3) Verification of adhesion protein expression of M2 macrophage-derived EVs

The degree of M2 polarization was analyzed by RT-qPCR for the mRNA expression of M2-specific macrophage markers (CD163, CD169, STAT6 VEGFA) and adhesion molecules (VCAM1, MAEA) that form the macrophage-erythroblast island. Primers of the macrophage markers and erythroid markers used in the analysis are shown in Table 1 below.

**[Table 1]**

| **Genes** | **primers** | **Sequences** | **SEQ ID NO:** |
|---|---|---|---|
| GAPDH | Forward | ccaacgtgtcagtggtggac | 1 |
| | Reverse | cctgcactttttaagagccagtc | 2 |
| EMP(MAEA) | Forward | gaagctcagcgtcctcaaga | 3 |
| | Reverse | ctggtcgctgctatgctct | 4 |
| VCAM1 | Forward | ccacagtaaggcaggctgta | 5 |
| | Reverse | ggaacaggtcatggtcacag | 6 |
| CD163 | Forward | gaagatgctggcgtgaca | 7 |
| | Reverse | gctgcctccacctctaag | 8 |
| CD169 | Forward | tgaggtggggaccagccgg | 9 |
| | Reverse | gtgaccaggacacacagcgg | 10 |
| STAT6 | Forward | tgaggtggggaccagccgg | 11 |
| | Reverse | gtgaccaggacacacagcgg | 12 |
| VEGFA | Forward | cacacaggatggcttgaaga | 13 |
| | Reverse | agggcagaatcatcacgaag | 14 |
| ICAM4 | Forward | cccttgtagggatcctcctc | 15 |
| | Reverse | ctcccaaagtgctgggatta | 16 |
| EPOR | Forward | catggatgaaggctcagaag | 17 |
| | Reverse | tgccagagtcagataccaca | 18 |
| AHSP | Forward | ttgaaggagttcagcgttct | 19 |
| | Reverse | tgtcacctgctgcctgtaat | 20 |

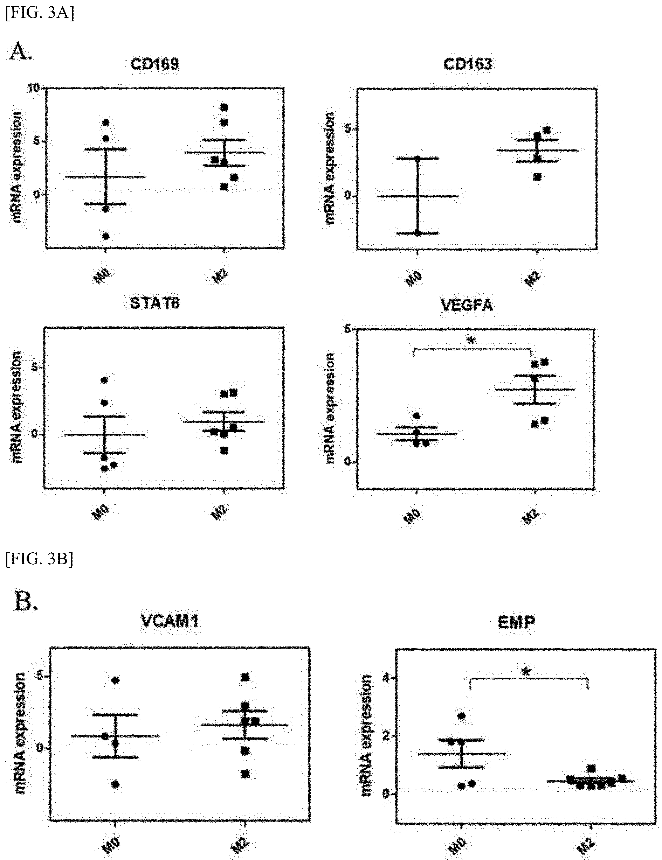

As a result of confirming the changes in the expression of the M2 markers (VEGFA, CD169, CD163, and STAT6), it was confirmed that VEGFA significantly increased 2.54-fold, and CD169 and STAT6 were expressed (n=4-5, student t-test; *p<0.05). It was observed that the M2 surface marker, CD163, was not detected at M0, but improved at M2. That is, it was confirmed that the macrophages appropriately differentiated into the M2 form (FIG. 3A). In addition, as specific adhesion-associated factors of erythroid precursor cells and macrophages in the erythroblastic island, it can be seen that adhesive molecules VCAM1 and EMP (MAEA) were detected at the mRNA level, and they were maintained as adhesive molecules forming the erythroblastic island even after polarization (n=5-7, student t-test; *p <0.05). Therefore, it was determined that the donor cells of the EVs, macrophages, appropriately differentiated to M2 (FIG. 3B).

### 2. Verification whether M2 macrophage-derived EVs were internalized into erythroid precursor cells

The EVs were labeled with fluorescence, and 24 hours later, it was confirmed whether they were internalized into erythroid precursor cells by z-stacking using confocal laser scanning microscopy (CLSM).

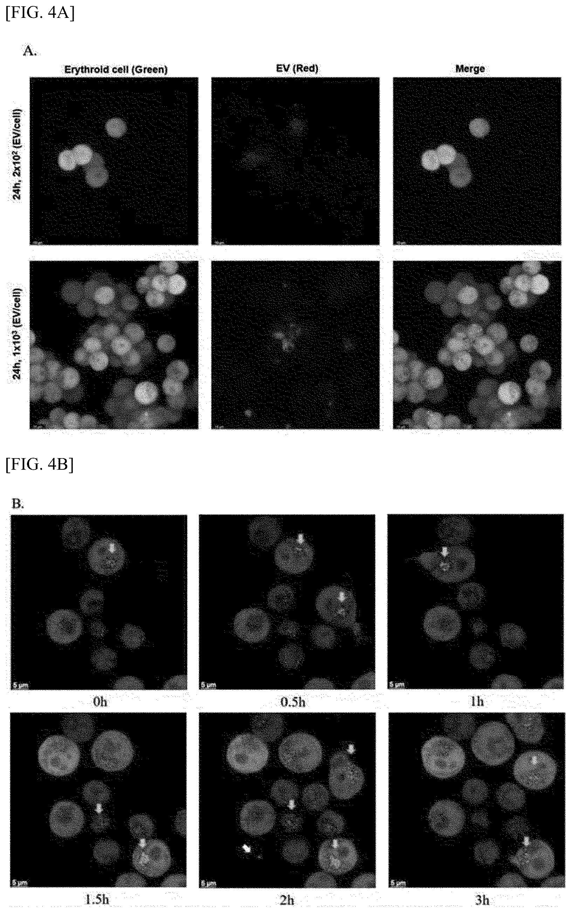

After isolated EVs were stained with an exosome-specific dye, to confirm whether the EVs were internalized into the cells, measurements were performed using CLSM under normal proliferation medium conditions 24 hours after treatment with EVs at concentrations of 2 x 10² and 1 x 10³ EVs/cell, as compared with a control that was not treated with EVs. Red labeled EVs were internalized into erythroid precursor cells expressing GFP in a concentration-dependent manner, and the resulting cells were analyzed by z-stacking, confirming that the EVs were located in the cytoplasm and their concentration increased around the aggregated erythroid precursor cells (FIG. 4). These results show that EVs are internalized into erythroid precursor cells within a concentration ranging from 2x10² to 1x10³ (EVs/cell).

In addition, for 3 hours after injection of EVs, the increase in expression of the labeled fluorescence due to the migration of erythroid precursor cells and the internalization of EVs were observed using CLSM live cell imaging. Specifically, using CLSM-based live cell imaging, after injection at 1 x 10³ EVs/cell 1 hour before measurements, z-stack images were acquired every 5 minutes for 3 hours, thereby observing erythroid precursor cells that migrate and interact with each other over time in a stationary state. Here, red-labeled EVs were localized in the cytoplasm, and their intensity increased over time. During the 3-hour observation, after about 1 hour, the erythroid-lineage cells showed a clustering pattern in a number increased 1.5-fold that in the initial frame.

### 3. Co-culture of erythroid precursor cells and macrophage-derived EVs

Erythroid precursor cells were tested under proliferation medium conditions and differentiation medium conditions. Under the proliferation medium conditions, the cells were cultured in a StemSpan^{™} (STEMCELL Technologies) medium supplemented with 1% penicillin-streptomycin (Gibco), 3 IU/mL EPO (Calbiochem, San Diego, CA), 50 ng/mL SCF (R&D Systems, Minneapolis, MN), 1 µM DEX, and 1 µg/mL doxycycline (Takara, Shiga, Japan). And a control without EV treatment and experimental groups treated with EVs at concentrations of 1 x 50, 1 x 10², and 2 x 10² EVs/cell were cultured for 30 days, with the differentiation medium being changed every 2 or 3 days. Likewise, under the same proliferation medium conditions, EVs were treated (0, 2 x 10², 1 x 10³, and 2 x 10³ EVs/cell) and then incubated for 24 and 72 hours.

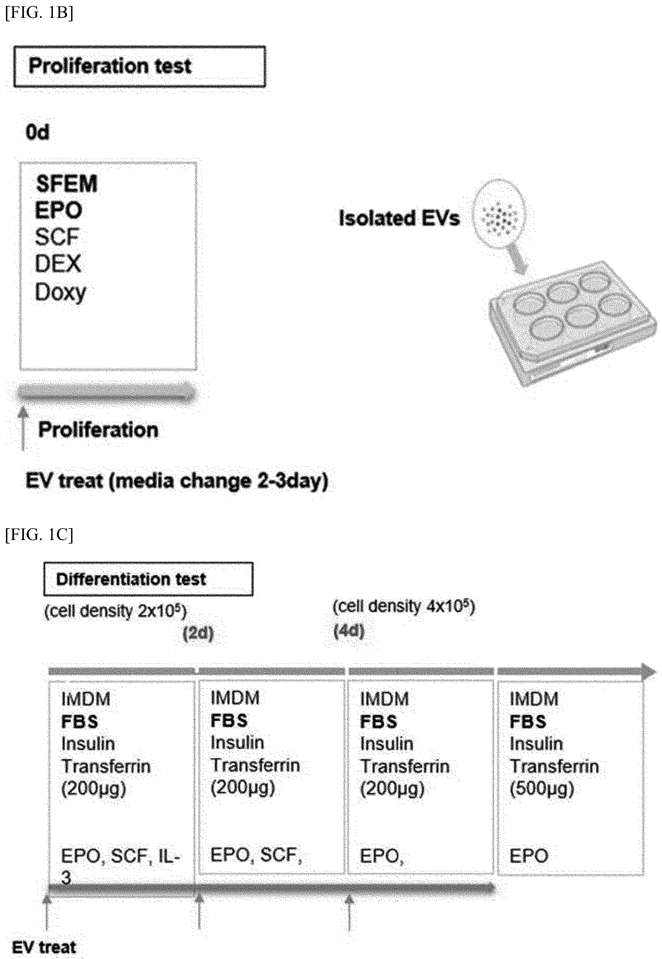

For the basal differentiation medium conditions, an Iscove's Modified Dulbecco's Medium (IMDM; Gibco, Waltham, MA) was prepared as a basal medium and supplemented with 10% Tet-free fetal bovine serum, 10 µg/mL insulin, 200 µg/mL transferrin, and 1% penicillin-streptomycin (Gibco), followed by filtering through a 20-µm filter. For 0 to 2 days, the basal medium was supplemented with cytokines (3 U/mL EPO, 10 ng/mL SCF, and 1 ng/mL IL3), and for 2 to 4 days, 3 U/mL EPO and 10 ng/mL SCF were added. The medium protocols used in proliferation and differentiation are shown in FIG. 1.

### 4. Verification of erythrocyte proliferation induced by M2 macrophage-derived EVs

### 1) Verification of proliferative tendency

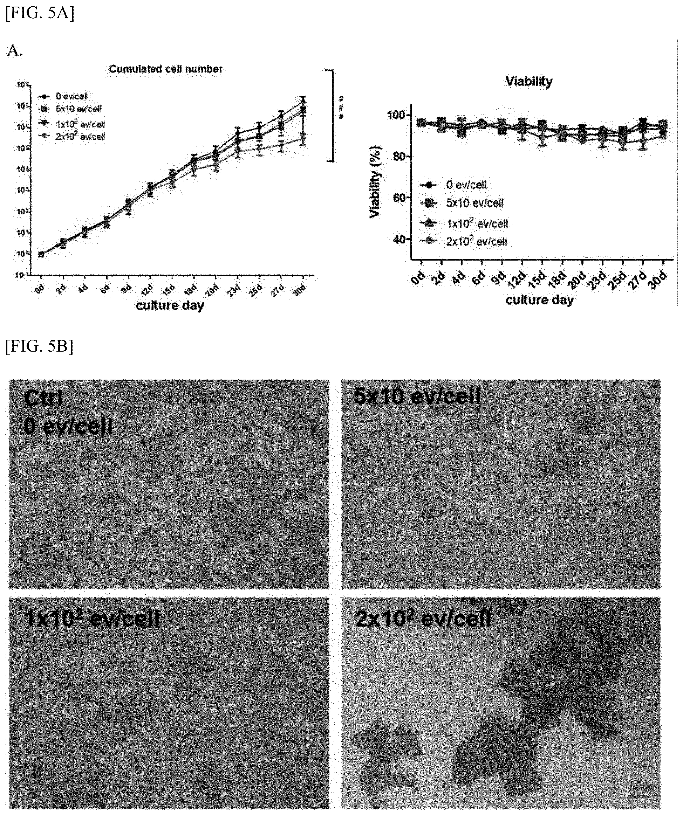

Immortalized human erythroid precursor cell lines were cultured in a proliferation medium for 30 days while being treated with different concentrations of EVs (0, 1x50, 1x10², and 2x10² EVs/cell). As a result, the higher the concentration of the EVs, the lower the cumulative cell number (FIG. 5A), and the aggregation of erythroid precursor cells, which was not shown during the initial sub-culture, was observed in the later stages of culture (FIG. 5B).

### 2) Toxicity evaluation

In addition, to evaluate the toxicity of macrophage-derived EVs, immortalized human erythroid precursor cell lines were cultured in a proliferation medium for 30 days, and treated with different concentrations of EVs (0, 1x50, 1x10², and 2x10² EVs/cell).

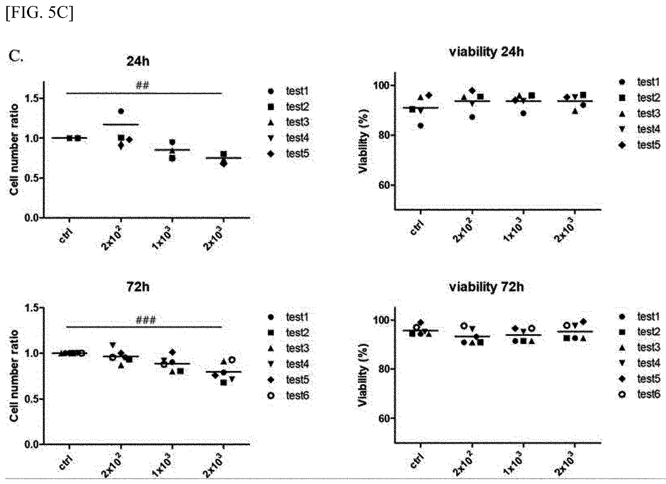

As a result, it was confirmed that viability is constantly maintained regardless of the concentration of EVs (FIG. 5C).

### 3) Verification of whether expression of cell adhesion molecules is increased by M2 macrophage-derived EVs

To verify whether macrophage-derived EVs cause the expression of cell adhesion molecules to increase, for 24 and 72 hours, after injection of EVs at different concentrations (0, 2x10², 1x10³, and 2x10³ EVs/cell), the expression patterns of erythroblastic island-associated adhesion proteins, EMP, ICAM4, EPOR, and AHSP, in erythroid precursor cells were examined through RT-qPCR.

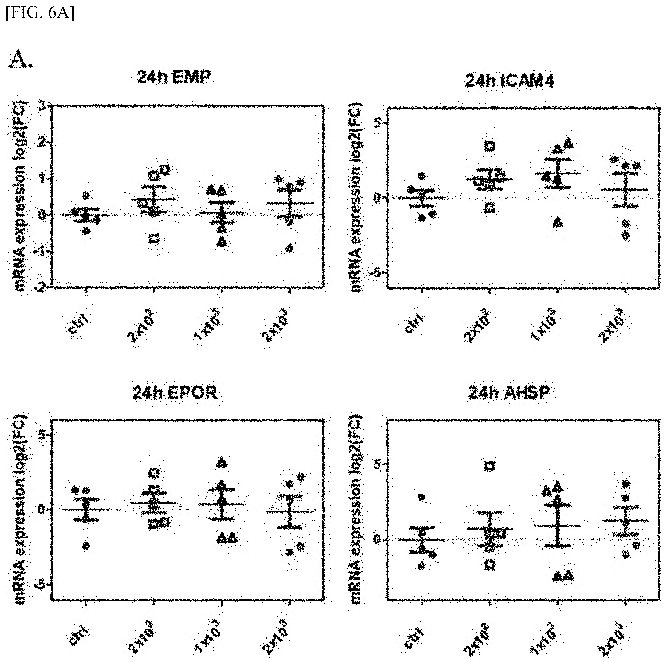

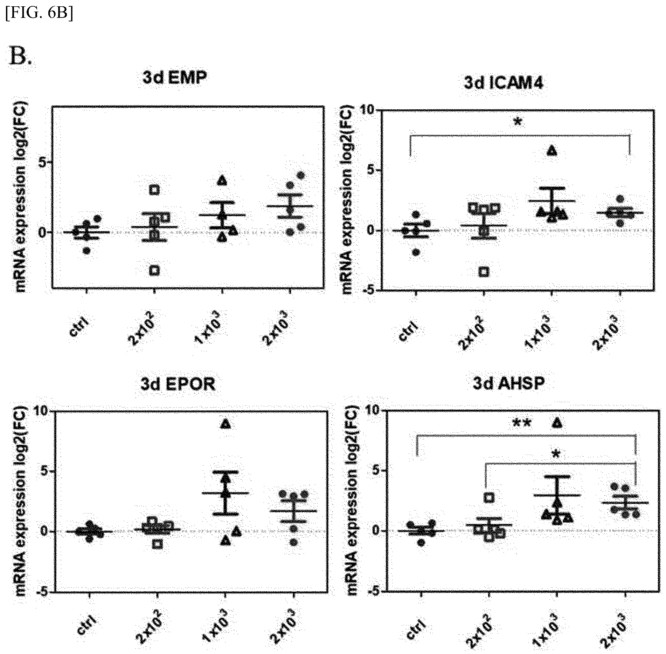

As a result, it was confirmed that, in the 24-hour treatment, the expression of the cell adhesion molecule ICAM4 increased 2.5-fold compared to the control at 2x10³ EVs/cell (FIG. 6A), and in the 72-hour treatment, the expression of AHSP, a gene specifically expressed in erythroid-lineage cells, increased 6-fold compared to the control at 2x10³ EVs/cell. In addition, it was confirmed that, at 2x10³ EVs/cell, rather than 2x10² EVs/cell, the AHSP expression increased significantly by 3.15-fold (FIG. 6B).

### 5. Verification of induction of erythroid precursor cell differentiation by M2 macrophage-derived EVs

### 1) Verification of protein (GPA and CD71) expression in erythroid precursor cells by M2 macrophage-derived EVs

EVs were treated at different concentrations (0, 2x10², and 1x10³) in an erythroid precursor cell differentiation medium to allow erythroid precursor cells to differentiate into erythrocytes. The enucleation rate of each sample of the EV-treated erythroid precursor cells and the protein (GPA and CD71) levels expressed in mature erythrocytes were assessed through flow cytometry and Wright-Giemsa staining. The results on day 2 and day 4 of differentiation were confirmed.

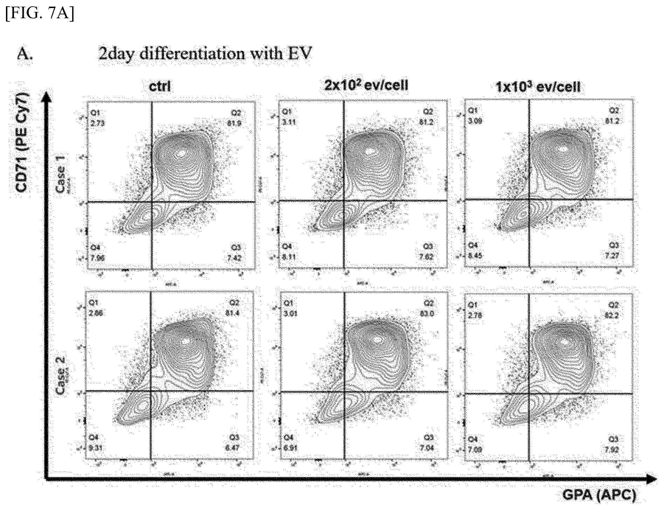

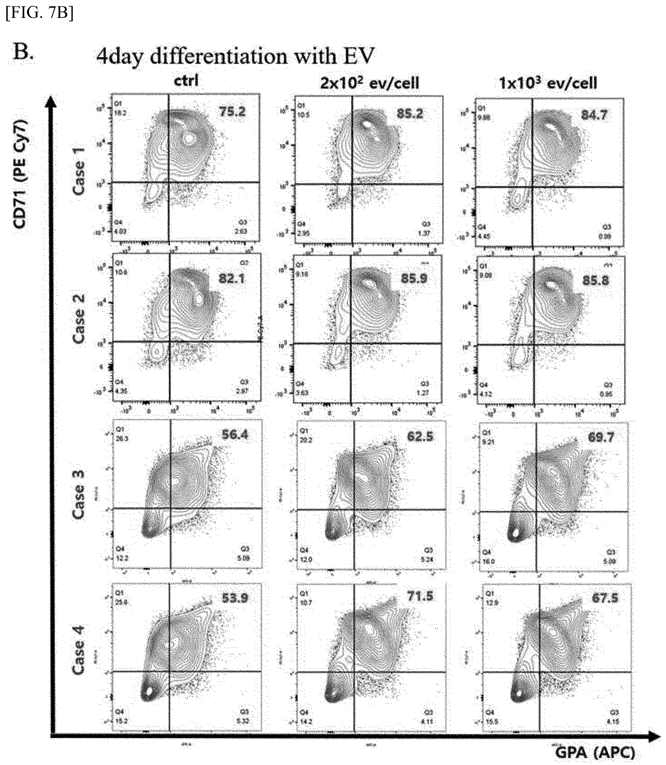

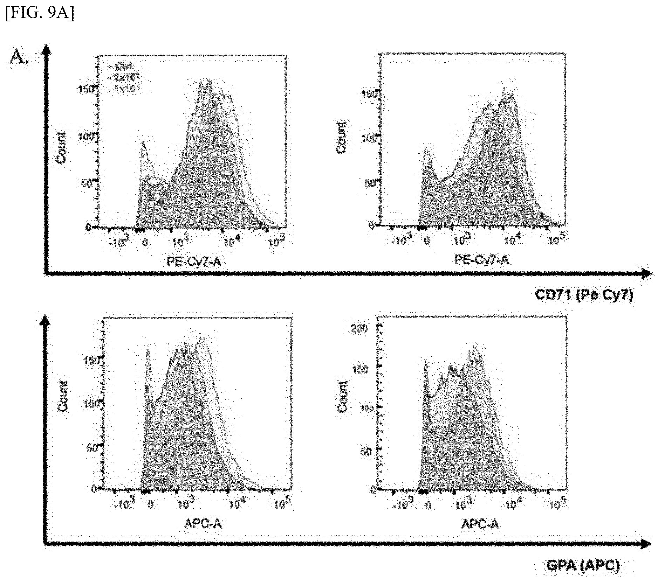

First, as a result of confirming the GPA and CD71 expression in the control and the EV-treated experimental groups, on day 2 of differentiation, there was no difference in expression of erythroid cell markers, GPA and CD71 (FIG. 7A), and on day 4 of differentiation, the average value of the expression of the GPA and CD71 markers increased 10% in the experimental groups treated with EVs (FIG. 7B), and when converted to mean fluorescence intensity (MFI) histograms, these increases were also observed in the experimental groups (FIG. 9A). That is, it was confirmed that the M2 macrophage-derived EVs aided the expression of specific markers in mature erythrocytes before enucleation.

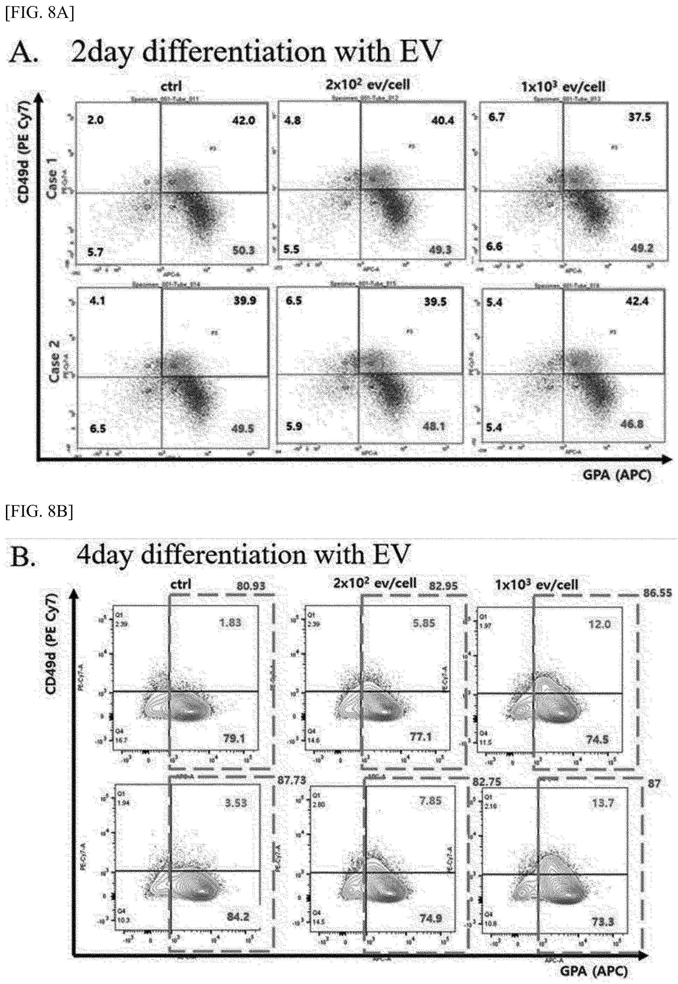

On the other hand, for CD49d whose expression decreases with differentiation into mature erythrocytes, no difference was observed between the control and the experimental groups on day 2 of differentiation (FIG. 8A). However, on day 4 of differentiation, the GPA(+)/CD49d(+) ratio was high in the EV-treated experimental groups (FIG. 8B). This result suggests that overall erythrocyte-specific GPA expression was relatively high and intense in the experimental groups, whereas the proportion of immature erythroblasts was also high.

### 2) Confirmation of enucleation in erythroid precursor cells by M2 macrophage-derived EVs

In addition, as a result of comparing the enucleation rates in the control and the EV-treated experimental groups, it was confirmed that, on day 2 of differentiation, there were no significant changes in enucleation rate between the control and the EV-treated experimental groups, but on day 4 of differentiation, the proportion of polychromatic erythroblast-orthochromatic erythroblasts increased in the experimental groups compared to the control.

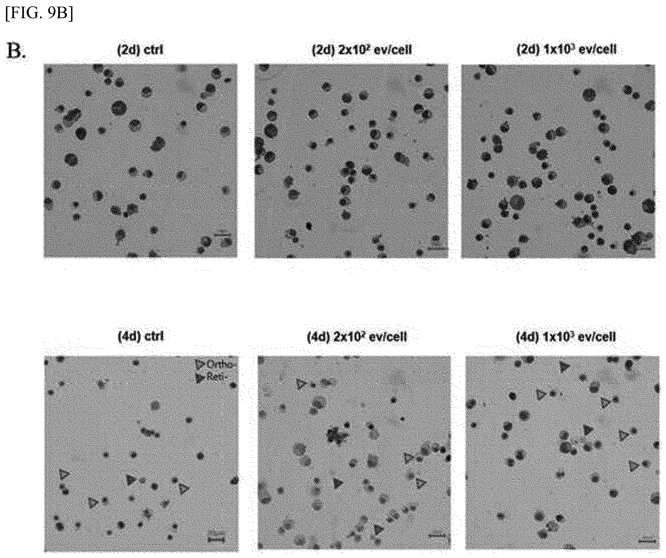

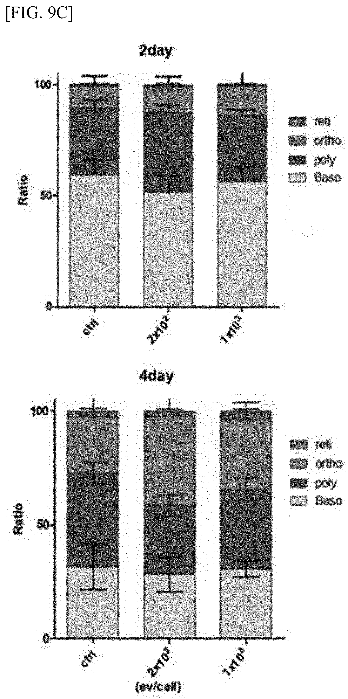

Specifically, on day 2 and day 4 of differentiation after Wright-Giemsa staining, morphology and maturation status was compared (FIG. 9B). While there was no difference between the experimental groups on day 2 of differentiation, the basophilic erythroblast proportions in the EV-treated experimental groups were similar to that of the control on day 4 (control: 31.73%, 2 x 10² EVs/cell: 28.33%, 1 x 10³ EVs/cell: 30.70%). Meanwhile, regarding the proportion of polychromatic erythroblasts, the control showed 41.01%, which was higher than the experimental groups (2 x 10² EVs/cell: 30.34%, 1 x 10³ EVs/cell: 35.04%). However, regarding the orthochromatic erythroblast proportion, the control showed 24.58%, which was lower than the EV-treated experimental groups (2 x 10² EVs/cell: 39.04%, 1 x 10³ EVs/cell: 30.45%) (FIG. 9C).

The present invention has been described with reference to the above-described examples and experimental examples, but this is merely illustrative, and it should be understood by those of ordinary skill in the art that various modifications and equivalent examples and experimental examples can be made therefrom. Therefore, the actual scope of the present invention should be determined from the technical spirit of the accompanying claims.

## Claims

1. A composition for use in inducing differentiation of erythroid precursor cells, comprising macrophage-derived extracellular vesicles.

2. The composition for use of claim 1, wherein the macrophage-derived extracellular vesicles promote the differentiation of erythroid precursor cells into erythrocytes.

3. The composition for use of claim 1, wherein the macrophages are M2 macrophages.

4. The composition for use of claim 1, wherein the macrophage-derived extracellular vesicles are directly internalized into the erythroid precursor cells.

5. The composition for use of claim 1, wherein the composition increases the expression of ICAM4 and/or AHSP in the erythroid precursor cells.

6. The composition for use of claim 1, wherein the composition increases the expression of GPA and/or CD71 in the erythroid precursor cells.

7. The composition for use of claim 1, wherein the diameter of the macrophage-derived extracellular vesicles ranges from 50 to 1000 nm.

8. The composition for use of claim 1, wherein the macrophage-derived extracellular vesicles are contained in the composition at a concentration of 1 x 10¹ to 1 x 10⁴ EVs/cell.

9. The composition for use of claim 1, wherein the composition further comprises EPO, SCF, or IL-3.

10. The composition for use of claim 1, wherein the erythroid precursor cells are selected from the group consisting of hematopoietic stem cells, burst-forming unit-erythroids (BFU-Es), colony-forming unit-erythroids (CFU-Es), proerythroblasts, basophilic erythroblasts, polychromatic erythroblasts, orthochromatic erythroblasts, and reticulocytes.

11. A method of inducing erythroid precursor cell differentiation, comprising culturing erythroid precursor cells in a medium containing macrophage-derived extracellular vesicles.

12. The method of claim 11, further comprising additionally treating EPO, SCF, or IL-3.
